# EUROPEAN PATENT APPLICATION

(11) **EP 0 819 388 A1**
(43) Date of publication of application: **21.01.1998**
(21) Application number: 96202012.9
(22) Date of filing: 16.07.1996
(51) Int. Cl.: A23L 3/3454, A23L 3/3409, A01J 7/04

(54) **Apparatus for disinfection in the alimentary industry, comprising an ozonized air generator**

(71) Applicant: GILNA CORPORATION N.V., Willemstad, Curaçao (AN)
(72) Inventor: Darecchio, Annibale, 43015 Noceto-Parma (IT)
(74) Representative: Perani, Aurelio

(57) **Abstract**

The invention concerns an apparatus for disinfecting the surface of products and objects of general use in the alimentary industry.

This apparatus comprises a container (1), where the product or object to be disinfected is accommodated and means (3) for producing ozone in an air flow intaken in the apparatus; the mixture of air and ozone is conveyed to the interior of the container (1) so that the product or object located therein is licked and disinfected by the ozone.

In an embodiment of the apparatus, the mixture of air and ozone is dispersed in water flowing toward the container, so that also a washing effect is provided by the invention.

The apparatus is particularly suitable for disinfecting teats (2) of milking animals.

## Description

The present invention relates to an apparatus for disinfection in the alimentary industry.

Before proceeding in this description, it matters to specify that within the meaning of the alimentary industry herein considered there are encompassed all the various industrial activities which are carried out along the chain of steps that brings foodstuffs in general, from their origin to the final consumers: these activities may thus include the packaging, the provisioning under different forms of raw products such as fruits, vegetables, meat, fish and so on, as well as their successive processing and transformation until the final condition under which they are market.

As a consequence, also the items whose surface is to be disinfected by the apparatus according to the invention may vary considerably in connection with the industrial activity to which reference is made, as it will be better appreciated below; it is further appropriate to specify that the apparatus of this invention is intended not only for foodstuffs, but also for objects of general use with which the latter may be brought into contact along the aforesaid chain, such as vessels or other various containers, working tools and so on.

It is well known that in many industrialized countries there exist standards rather severe for ruling the sanitary conditions required during the different phases in which the production cycles of the alimentary industry are articulated; for better appreciating this fact, either in this preliminary part of the description and in the embodiments of the invention which will be considered later reference will be made to the dairying: however the arguments which will the put forward should be kept valid also for other alimentary industrial fields, where appropriate with adequate adaptations.

In the light of this foreword in the dairying for obtaining high quality milk from a microbiological point of view and thus preventing relevant economical losses due to the waste of amounts of milk that do not fulfil the sanitary requirements, it is necessary to avoid the so called microbic pollution of the raw material: this applies either to the milking phase, either to that of transport of the milk, as well as to the various transformations of it in final products (cheese, yoghurt, etc.).

As regards milking, normally the surface of the udders and especially that of their teats is contaminated by fecal residues or milk encrustations deriving from previous milking; such residues and particularly the micro-organisms contained therein, during milking commonly pass in the milk there by contaminating it.

This fact may cause serious economical damages especially for cheeses which must be produced without pasteurizing the milk; indeed, the existence in the milk of harmful micro-organisms is not acceptable beyond predetermined levels foreseen by the sanitary standards referred to above and as a consequence the stocks of milk contaminated beyond those levels, as well as the products deriving therefrom, cannot be marketed with evident economical losses for the manufacturers.

In several, small farms usually the above mentioned residues are manually removed before starting the milking phase; such manual removal of the residues is however not satisfactory, especially if carried our in dry conditions, that is to say without using washing liquids.

From a few time there have been developed and are used with success portable devices provided with rotating brushes, acting on a teat to be washed together with water conveyed in the zone where the brushes operate; these devices allow to achieve rapid washing which is better than the manual one, and provide for limiting the milk contamination below 100.000 bacteria per cm³.

For further enhancing the performances of these rotating brushes devices, in place of normal water generally used sometimes there are employed solutions of water with detergent and/or antiseptic substances; such solutions leave however chemical residues on the teats of the animals, which may contaminate the milk thereby rendering unuseful or at least limiting the effectiveness of the devices.

For information, the more commonly used antiseptic substances are hydrogen peroxide, quaternary ammonium bases, some iodine compounds, the chlorohexidine ((1,6-bis-p-chloro-phenyl-diguamido)hexane).

Another possible source of milk bacteric pollution is the surface of the objects with which the milk comes into contact during the cycle that brings it from the milking phase through its transformations, to the final packaging for the market: these objects may be the vessels for collecting and transporting milk, the tools for mixing it and any other possible one; generally, the surface of these objects is washed with normal water or with a detergent and/or antiseptic solution, so as to incur again in the same drawbacks already outlined above.

Generally speaking, in different fields of the alimentary industry there are used several techniques for disinfecting the surface of the aliments or of the objects used in the productive cycle, among which there are hereby mentioned: the pasteurization, the thermal sterilization and the so called cold sterilization achieved by means of electromagnetic radiations such as gamma rays, x-rays, ultra-violet rays or other kinds of radiations, treatments with chemical agents such as alcohol, aqueous chlorine solutions or other substances.

Each of these techniques, however, has some drawbacks that are generally involved by their conditions of application.

For instance some of them operate only at high temperatures or in any case not at ambient temperature, which fact is not acceptable in some applications: reference can be made to the case above of disinfection of teats of milking animals, that preferably should be carried out at ambient temperature or however at the most with tepid water.

In the case of irradiation with gamma, X or ultra-violet rays, the disinfection should be carried out on surfaces that are not handled by persons and anyhow adequate protecting means shall be provided; it must also be taken into account the fact that the use of irradiation needs generally the supervision of specialized personnel and this fact renders less favourable the use of these techniques on an industrial scale.

The use of chemical substances for disinfecting surfaces has the relevant drawback that possible residual traces formed on the surfaces may have highly negative effects on the taste of the alimentary products.

In short it can be said that the known techniques of disinfection and the apparatuses for their application, do not have generally sufficient flexibility and versatility so as to be used efficaciously during the different production processes inherent the alimentary industry: in other terms, it has been verified that each technique of disinfection may be employed in a limited number of industrial applications. This fact occurs either because the apparatuses needed for applying such techniques are rather complex, either because their conditions of operation do not fit with the requirements of industrial production cycles which become more and more rapid in the modern alimentary industry, or finally because they are not suitable for being applied on living animals or parts thereof such as in the case of teats of milking cattle.

The technical problem underlying this invention is that of providing an apparatus for carrying out the disinfection of surfaces in the alimentary industry, which is able to overcome the drawbacks cited in connection with the prior art.

This problem is solved by an apparatus whose features are defined in the appended claims.

For a better understanding of the present invention as a whole, its features and the advantages achieved, there will be hereby described two indicative and non-limitative embodiments thereof, shown in the annexed drawings wherein:
- figure 1 shows schematically a first embodiment of an apparatus according to the invention;
- figure 2 shows schematically a second embodiment of the apparatus of this invention.

With reference to the first of the figures listed above, an apparatus according to the invention includes a spout 1 of the type used for milking animals, which is applied to a teat indicated by reference numeral 2; the apparatus further comprises an electrical devise 3 for producing ozone in the air for instance by means of electrical discharges or of special radiant lamps.

Moreover, in this embodiment there is a mixer 5 for mixing water with air containing a fraction of ozone produced by the device 3 above; such mixer basically consist of an ejector comprising a Venturi tube and a nozzle coaxial to it from which ejects, at high speed, water which may arrive from the normal civil delivery system; in the narrowing part of the Venturi tube the speed of the water causes a depression that intakes a flux of air wherein ozone has been produced by the device 3, which air is then dispersed in the water under the form of micro-bubbles.

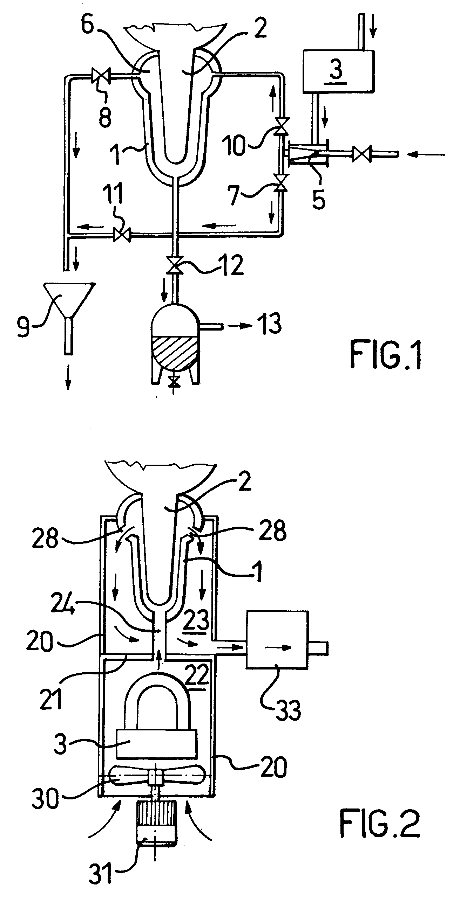

For the operation of the apparatus there are also provided electrical solenoid valves 7,8,10,11 and 12 that control the flux of liquids along a network of flexible pipes, shown in figure 1, connected further than to the outlet of the mixer 5, also to a dump of exhaust washing liquids 9 and to a milking device 13; a microprocessor not shown in the drawings co-ordinates and controls the operation of the apparatus, determining the switching in the open or close position of the valves according to an operative cycle set out below.

Such operative cycle of the teat 2 is carried out in two steps, making water containing bubbles of air and ozone to flow in the space 6 defined between the spout and the teat 2.

During the first step the ozonized water, that is to say water coming out from the mixer 5 and wherein bubbles of the mixture formed by air and ozone are dispersed, so that the teat is washed from bottom to top thereby removing the possible solid residues deposited on it. The flowing of ozonized water coming out from mixer 5, is achieved in this first step by opening valves 7,8 and closing valves 10,11.

In the second step of the cycle the ozonized water enters into the upper part of spout 1 and comes out from the lower one thereof, dragging with it toward dump 9 the residues removed during the first step; in this circumstance the circulation of the liquids within the apparatus is achieved by closing valves 7,8 and opening valves 10,11.

After this cycle of washing and disinfection it is possible to start the milking; this is made by closing valves 10,11 and opening valve 12 which connects the apparatus of the invention to the making device 13.

From the example just described it is possible to appreciate how the present invention solves the technical problem underlying it: this result is achieved in view of the contact of the surface to be disinfected, in this case the teat 2, with the ozone dispersed in the water circulating in the apparatus. In fact it has been possible to verify that the ozone dispersed in the water is able to decontaminate the surface of the teat from which possible scales of solid deposits have been removed because, otherwise, they could prejudice the disinfecting action.

It has to be pointed out that in the apparatus according to this invention, the ozonized water does not leave residual traces on the teat or on the inner wall of the spout thus avoiding the risk of contaminating afterwards the milk, because the ozone contained in it dissolves after a short lapse of time transforming itself in molecular oxygen, that is a gas which is a natural component of air; it is also appropriate to observe that the working temperature for the apparatus just described is the ambient temperature and therefore such apparatus may be favourably used on animals, even if it shall not be excluded the use of warm water.

Moreover, in the specific context of dairying it should be taken into account that the substances present in the milk such as butterfat, proteins and vitamins do not undergo alterations for the fact that the disinfection temperature is the ambient one; this applies above all in the case of disinfection of objects or containers with which the milk may be brought into contact during the manufacturing process.

It must also not be overlooked the advantageous effect obtained by the apparatus of the invention, according to which for disinfecting a surface no chemical products need be used: this avoids to store, handle or dose such products, and all the inconveniences that may derive therefrom: reference can be made for instance, to the case of disinfection of teats of cattle and to the problems that could arise for handling chemical products in some farms or cowsheds.

Amongst the other advantages achieved by this invention is its constructional and functional simplicity, deriving also from the fact that for the devices for producing ozone, commercially available models may be used with low costs and low energy consume because they can work with the electric energy delivered for civil uses; the devices for producing ozone are, as stated before, preferably of the type operating with electrical discharges or special radiant lamps.

In a preferred embodiment, it is also provided that during operation of the apparatus of the invention there is no need of adjusting the ozonization intensity of the air carried out by the device 3, since the same can be pre-set in connection with the kind of use to be made: for instance, such an intensity may vary depending on the surface to be disinfected that can be an anatomical part of an animal like the teat before or an object, as could be the case of the spout or of a vessel.

In other terms, it must be underlined that in the apparatus of the invention there is the possibility of setting it by controlling some working parameters, such as the ozone amount to be produced in the air intaken, the flow rate of such air or of the water fed to the mixer 5 and so on, in order to adapt it to different circumstances. Therefore these apparatuses can be used either for disinfecting teats of cattle or more generally anatomical tissues that need a low ozone production which avoids alterations of them such as the hardening of the teat, either for disinfecting objects of general purposes to which a greater quantity of ozone may be applied.

It can be appreciated that persons skilled in the art may find variants to the embodiment of the apparatus previously described, also in view of the fact that the present invention can be applied to different fields of the alimentary industry and not only to dairying; the description of one possible variant is hereby reported with reference to figure 2 wherein the same reference numerals indicate the parts of the invention structurally or functionally equivalent to those already referred to above.

In such a variant, the apparatus is provided with a case 20 internally divided by a partition 21 in two parts 22 and 23; across the partition 21 there extends a conduit 24 which makes the first part 22 of the case 20 to communicate with the interior of the spout 1 wherein it is accommodated a teat 2. The spout 1 is provided with a series of openings 28 adapted to allow the outlet of a fluid, in this example a mixture of air and ozone, as shown by arrows in the drawings and explained hereinafter.

In the first part 22 of the case 20 there are also contained means 3 for producing ozone, in this case consisting of a radiant lamp, adjacent to which there is a fan 30 driven by a motor 31; associated to the second part 33 of the case 20 there is a de-ozonizer device 33, comprising a special lamp different from the radiant one above. Such device 33 could however be also of chemical type, for instance based on the use of appropriate catalysts.

In this variant of the invention, the first part 22 of the case 20 communicates with the exterior in order to intake air following the operation of the fan, whilst the second part 23 when a teat 2 is accomandated in the spout 1 communicates with the exterior only through the outlet of the de-ozonizer device 33. In practice during operation of the apparatus a fluid circulation occurs as follows.

The case 20 is applied to a teat 2 previously washed so as to remove possible solid residues on the latter, by introducing it in the spout 1; it should be observed that for this purpose on the case 20 a handle not shown in the drawings, could be provided. Afterwards the radiant lamp 3 is turned on together with the fan so that an air and ozone flow is conveyed from the first part 22, through the conduit 24, toward the interior of the spout 1; the mixture of air and ozone that arrives there licks the teat 2 thereby disinfecting it and then comes out of the spout 1, through the openings 28, into the second part 23 of the case 20.

From here the mixture passes through the de-ozonizer device 33 and thus arrives to the exterior of the apparatus.

In this embodiment of the invention therefore, the ozone mixed with air is not dispersed, under the form of bubbles, in a liquid which serves for its conveying but is directly applied on the surface to be disinfected; it has to be observed that whereas in the first embodiment the water allows the remaining ozone to be decomposed thus avoiding that such gas comes out of the apparatus, in this variant there has been foreseen an appropriate device 33 for eliminating the remaining ozone: this has been made for conferring greater security to the persons who handle the apparatus, because the possible contact with the eyes, face, skin and eventually also the breathing of the ozone may be harmful.

As above stated, the apparatus in accordance with the invention may be applied in different fields of the alimentary industry and not only to dairying or milking; therefore it can be understood that it should not be excluded that the apparatus might undergo relevant changes in order to be adapted to different products or objects that concern each alimentary field; for example the spout 1, which in substance is an element for accommodating the body to be disinfected, could be replaced by containers of various forms and dimensions.

Generally speaking it can be said that the apparatus of the invention in view of its simplicity, concerning either the structural and functional aspects, is particularly versatile and useful for fulfilling different necessities: depending on the uses to be made, it may be designed either according to a portable model, at least as regards its part associated to the container element such as the case of the spout above, either to a fixed configuration.

## Claims

1. Apparatus for the disinfection in the alimentary industry, characterized in that it comprises a container (1) adapted to accommodate at least one body (2) to be disinfected, means (5; 30,31) for generating an air flow intaken from the exterior of the apparatus toward the latter, a device (3) for producing ozone in such air flow, means (5,7,8,10,11 and 12; 24,30) for conveying a fluid that comprises the air with the ozone produced therein by the aforesaid device (3) or at least a fraction thereof, to the interior of the container (1) wherein the surface of said at least one body (1) is licked by the fluid and disinfected by the ozone contained therein.

2. Apparatus according to claim 1, characterized in that the means for conveying the fluid comprise a mixer (5) wherein the air with the ozone contained in it are dispersed as bubbles in a liquid, and in that the aforesaid fluid consists of such liquid with the bubbles dispersed in it.

3. Apparatus according to claim 2, wherein the liquid is water.

4. Apparatus according to one of claims 2 or 3, characterized in that the mixer (5) substantially consists of an ejector comprising a Venturi tube wherein the aforesaid liquid is fed thereby causing the air flow intaken from the exterior of the apparatus.

5. Apparatus according to claim 1, characterized in that the means for conveying the fluid comprise fan (30, 31) adapted to intake air from the exterior of the apparatus thereby causing also the air flow wherein the ozone is produced, and in that the fluid conveyed to the container (1) consists of a mixture of the intaken air and the ozone produced therein or at least a part thereof.

6. Apparatus according to claim 5, characterized in that it comprises a de-ozonizer device (33) communicating with the container (1), wherein said mixture conveyed to the container flows downstream of it and the remaining ozone is decomposed.

7. Apparatus according to claim 6, characterized in that the de-ozonizer device is of electrical type and includes an appropriate lamp.

8. Apparatus according to claim 6, characterized in that the de-ozonizer device (33) is of chemical type based on the use of catalysts.

9. Apparatus according to any of the preceding claims, characterized in that the device (3) for producing ozone in the air flow intaken, is of the type operating with electrical discharges.

10. Apparatus according to any of the claims from 1 to 8, characterized in that the device (3) for producing ozone includes a radiant lamp.

11. Apparatus according to any of the claims from 1 to 8, characterized in that the container (1) comprises at least one spout of the type used for milking animals.

12. Apparatus according to claim 11, characterized in that it comprises means (7,8,10,12) adapted to make said at last one spout communicating with a device (13) for milking.
